# FASCICULE DE BREVET EUROPEEN

(11) **EP 4 558 265 B1**
(45) Date de publication et mention de la délivrance du brevet: **01.07.2026**
(21) Numéro de dépôt: 23739579.3
(22) Date de dépôt: 11.07.2023
(51) Int. Cl.: B01J 21/04, B01J 23/755, B01J 23/72, B01J 35/60, B01J 37/02, B01J 37/08, B01J 37/18

(54) **PROCEDE DE PREPARATION D'UN CATALYSEUR COMPRENANT UNE PHASE ACTIVE DE NICKEL ET UN ALLIAGE NICKEL CUIVRE**
VERFAHREN ZUR HERSTELLUNG EINES KATALYSATORS MIT EINER AKTIVEN NICKELPHASE UND EINER NICKEL-KUPFER-LEGIERUNG
METHOD FOR PREPARING A CATALYST CONTAINING AN ACTIVE NICKEL PHASE AND A NICKEL-COPPER ALLOY

(30) Priorité: 21.07.2022 FR 2207484
(43) Date de publication de la demande: 28.05.2025
(73) Titulaire: IFP Energies nouvelles, 92500 Rueil-Malmaison (FR)
(72) Inventeur: BOUALLEG, Malika, 92852 RUEIL-MALMAISON CEDEX (FR); JOTHIE, Laetitia, 92852 RUEIL-MALMAISON CEDEX (FR)
(74) Mandataire: IFP Energies nouvelles
(86) Numéro de dépôt international: PCT/EP2023/069134
(87) Numéro de publication internationale: WO 2024/017703

(56) Documents cités:
- WO-A1-2014/128203
- WO-A1-2019/201617
- WO-A1-2021/018599

## Description

### Domaine technique

La présente invention concerne un catalyseur métallique supporté à base de nickel et de cuivre destiné particulièrement à l'hydrogénation des hydrocarbures insaturés, et plus particulièrement, d'hydrogénation sélective de composés polyinsaturés ou d'hydrogénation des aromatiques.

### Etat de la technique

Les catalyseurs d'hydrogénation sélective de composés polyinsaturés ou d'hydrogénation des aromatiques sont généralement à base de métaux du groupe VIII de la classification périodique des éléments tel que le nickel. Le métal se présente sous la forme de particules métalliques nanométriques déposées sur un support qui peut être un oxyde réfractaire. La teneur en métal du groupe VIII, la présence éventuelle d'un deuxième élément métallique, la taille des particules de métal et la répartition de la phase active dans le support ainsi que la nature et distribution poreuse du support sont des paramètres qui peuvent avoir une importance sur les performances des catalyseurs.

La vitesse de la réaction d'hydrogénation est gouvernée par plusieurs critères, tels que la diffusion des réactifs vers la surface du catalyseur (limitations diffusionnelles externes), la diffusion des réactifs dans la porosité du support vers les sites actifs (limitations diffusionnelles internes) et les propriétés intrinsèques de la phase active telles que la taille des particules métalliques et la répartition de la phase active au sein du support.

En vue d'obtenir de meilleures performances catalytiques, notamment une meilleure sélectivité et/ou activité, il est connu dans l'état de la technique de procéder à l'utilisation d'additifs de type composés organiques pour la préparation de catalyseurs métalliques d'hydrogénation sélective. Par exemple, la demande FR2984761 divulgue un procédé de préparation d'un catalyseur d'hydrogénation sélective comprenant un support et une phase active comprenant un métal du groupe VIII, ledit catalyseur étant préparé par un procédé comprenant une étape d'imprégnation du support avec une solution contenant un précurseur du métal du groupe VIII et un additif organique, plus particulièrement un composé organique présentant une à trois fonctions acides carboxyliques, une étape de séchage du support imprégné, et une étape de calcination du support séché afin d'obtenir le catalyseur.

Par ailleurs, la promotion de catalyseur à base de nickel a fréquemment été proposée afin d'améliorer les performances en hydrogénation sélective. Par exemple, il est connu du brevet US 5,208,405 de mettre en œuvre un catalyseur à base de nickel et d'argent pour l'hydrogénation sélective de dioléfines en C4-C10. D'autre part, il est connu de promouvoir le nickel, présent majoritairement, avec des métaux du groupe IB, en particulier l'or (FR 2,949,077) ou l'étain (FR 2,949,078). Le document FR 3,011,844 divulgue un catalyseur pour la mise en œuvre d'un procédé d'hydrogénation sélective comprenant un support et une phase métallique active déposée sur le support, la phase métallique active comprenant du cuivre et au moins un métal de nickel ou de cobalt dans un ratio molaire Cu : (Ni et/ou Co) supérieur à 1.

Enfin, préalablement à l'utilisation de tels catalyseurs et leur mise en œuvre dans un procédé d'hydrogénation, une étape de traitement réducteur en présence d'un gaz réducteur est réalisée de manière à obtenir un catalyseur comprenant une phase active au moins partiellement sous forme métallique. Ce traitement permet d'activer le catalyseur et de former des particules métalliques. Ce traitement peut être réalisé in-situ ou ex-situ, c'est-à-dire après ou avant le chargement du catalyseur dans le réacteur d'hydrogénation.

Le document FR3099390A1 divulgue un catalyseur comprenant du nickel et du cuivre, à raison de 1 et 50 % en poids en élément nickel par rapport au poids total du catalyseur, à raison de 0,5 à 15 % en poids en élément cuivre par rapport au poids total du catalyseur, et un support d'alumine, ledit catalyseur étant obtenu par un procédé de préparation comprenant les étapes suivantes :
a) on met en contact le support d'alumine avec au moins une solution contenant au moins un précurseur de nickel ;
b) on met en contact le support d'alumine avec au moins une solution contenant au moins un précurseur de nickel et au moins un précurseur de cuivre ;
c) on met en contact le support d'alumine avec au moins une solution contenant au moins un composé organique comprenant au moins une fonction acide carboxylique, ou au moins une fonction alcool, ou au moins une fonction ester, ou au moins une fonction amide, ou au moins une fonction amine,
   étant entendu que :
   - les étapes a), b) et c) sont réalisées séparément, dans un ordre indifférent, ou
   - les étapes a) et c) sont réalisées simultanément, l'étape b) étant réalisée soit avant la combinaison des étapes a) et c), soit après ;
   - les étapes b) et c) sont réalisées simultanément, l'étape a) étant réalisée soit avant la combinaison des étapes b) et c), soit après ;
d) on réalise au moins une étape de séchage du précurseur de catalyseur obtenu à l'issue des étapes a) à c) à une température inférieure à 250°C ;
e) on réalise une étape de réduction du précurseur de catalyseur obtenue à l'issue de l'étape d) par mise en contact dudit précurseur avec un gaz réducteur à une température supérieure ou égale à 150°C et inférieure à 250°C.

Poursuivant ses recherches dans le domaine des catalyseurs d'hydrogénation sélective de composés polyinsaturés ou d'hydrogénation des aromatiques, la Demanderesse a maintenant identifié que l'on pouvait préparer un catalyseur particulièrement actif en réalisant un procédé de préparation spécifique dans lequel on ajoute une phase métallique à base de nickel et de cuivre en présence d'un additif organique particulier sur le catalyseur après le dépôt du précurseur de la phase active à base de nickel et en présence également d'un additif organique particulier.

Sans vouloir être lié par une quelconque théorie, il a été constaté par la Demanderesse que lors de la préparation du catalyseur, la réalisation d'une étape de mise en contact du catalyseur avec une solution contenant simultanément un précurseur métallique à base de cuivre, un précurseur métallique à base de nickel, et un additif organique particulier, suivie d'une étape de séchage et de réduction en présence d'un gaz réducteur à basse température (supérieure ou égale à 150°C et inférieure à 200°C) permet d'obtenir un alliage de nickel-cuivre (sous forme réduite) qui permet de manière inattendue d'améliorer fortement la réductibilité de la phase active de nickel sur le support, ladite phase active de nickel étant approvisionnée dans une étape antérieure à la formation de l'alliage de nickel-cuivre (sous forme réduite). Le procédé de préparation selon l'invention permet ainsi de réaliser une étape de réduction des éléments métalliques en présence d'un gaz réducteur à des températures plus basses et des temps de réaction plus court que ceux couramment utilisés dans l'art antérieur. Avantageusement, le recours à des conditions opératoires moins sévères que dans l'art antérieur permet de réaliser directement l'étape de réduction au sein du réacteur dans lequel on souhaite réaliser l'hydrogénation de composés aromatiques. Cependant l'ajout de nickel et de cuivre après l'ajout de la phase active de nickel conduit inexorablement au grossissement des particules de nickel et donc à une perte substantielle d'activité. C'est pourquoi la Demanderesse a identifié que l'ajout d'additif organique particulier, à des teneurs bien supérieures de celles utilisées lors de l'imprégnation de la phase active de nickel permet de limiter le grossissement des particules de nickel voir de l'éviter totalement.

### Objets de l'invention

La présente invention a pour objet un procédé de préparation d'un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, la taille des particules de nickel dans le catalyseur, mesurée sous forme oxyde, étant inférieure à 5 nm, lequel procédé comprend au moins les étapes suivantes :
a) on met en contact le support d'alumine avec au moins une solution contenant au moins un premier précurseur de nickel et au moins un premier composé organique comprenant au moins une fonction acide carboxylique pour obtenir un premier précurseur de catalyseur ;
b) on sèche le premier précurseur de catalyseur obtenu à l'issue de l'étape a) à une température inférieure à 250°C puis on calcine le premier précurseur de catalyseur séché à une température comprise entre 250°C et 550°C pour obtenir un précurseur de catalyseur calciné ;
c) on met en contact le précurseur de catalyseur calciné obtenu à l'issue de l'étape b) avec au moins une solution contenant au moins un deuxième précurseur de nickel, au moins un précurseur de cuivre et au moins un second composé organique comprenant au moins une fonction acide carboxylique pour obtenir un second précurseur de catalyseur ;
d) on sèche le second précurseur de catalyseur obtenu à l'issue de l'étape c) à une température inférieure à 250°C.

Selon un ou plusieurs modes de réalisation, le rapport molaire entre ledit composé organique introduit à l'étape a) et l'élément nickel également introduit à l'étape a) est compris entre 0,01 et 5,0 mol/mol.

Selon un ou plusieurs modes de réalisation, le rapport molaire entre ledit composé organique introduit à l'étape c) et l'élément nickel également introduit à l'étape c) est compris entre 0,02 et 5 mol/mol.

Selon un ou plusieurs modes de réalisation, le rapport molaire entre le nickel introduit lors des étapes a) et c) et le cuivre introduit lors de l'étape c) est compris entre 0,5 et 5 mol/mol.

Selon un ou plusieurs modes de réalisation, le ratio entre le rapport molaire entre le second composé organique et le nickel introduit à l'étape c) et le rapport molaire entre le premier composé organique et le nickel introduit à l'étape a) est supérieur à 1,5.

Selon un ou plusieurs modes de réalisation, les étapes a) et b) sont réalisées sont réalisées au moins deux fois avant de réaliser l'étape c).

Selon un ou plusieurs modes de réalisation, le premier composé organique de l'étape a) et le second composé organique de l'étape c) sont choisis parmi l'acide oxalique, l'acide malonique, l'acide glycolique, l'acide acide lactique, l'acide tartronique, l'acide citrique, l'acide tartrique, l'acide pyruvique, l'acide lévulinique.

Selon un ou plusieurs modes de réalisation, le premier composé organique de l'étape a) et le second composé organique de l'étape c) sont identiques.

Selon un ou plusieurs modes de réalisation, le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre et le fluorure de cuivre.

Selon un ou plusieurs modes de réalisation, le premier précurseur de nickel et/ou le deuxième précurseur de nickel est le nitrate de nickel, le chlorure de nickel, l'acétate de nickel ou l'hydroxycarbonate de nickel.

Selon un ou plusieurs modes de réalisation, ledit procédé comprend en outre une étape e) dans laquelle on calcine le catalyseur obtenu à l'issue de l'étape d) à une température comprise entre 250°C et 550°C.

Selon un ou plusieurs modes de réalisation, ledit procédé comprend en outre une étape f) dans laquelle on réduit le catalyseur obtenu à l'issue de l'étape d), optionnellement obtenu à l'issue de l'étape e), par mise en contact dudit catalyseur avec un gaz réducteur à une température supérieure ou égale à 150°C et inférieure à 250°C.

Un autre objet selon l'invention concerne un catalyseur obtenu par le procédé de préparation selon l'invention.

Un autre objet selon l'invention concerne un procédé d'hydrogénation sélective de composés polyinsaturés contenant au moins 2 atomes de carbone par molécule contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur selon l'invention ou obtenu selon le procédé de préparation selon l'invention.

Un autre objet selon l'invention concerne un procédé d'hydrogénation d'au moins un composé aromatique ou polyaromatique contenu dans une charge d'hydrocarbures ayant un point d'ébullition final inférieur ou égal à 650°C, ledit procédé étant réalisé en phase gazeuse ou en phase liquide, à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire comprise entre 0,05 et 50 h⁻¹, en présence d'un catalyseur selon l'invention ou obtenu selon le procédé de préparation selon l'invention.

### Description détaillée de l'invention

### 1. Définitions

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII (ou VIIIB) selon la classification CAS correspond aux métaux des colonnes 8, 9 et 10 selon la nouvelle classification IUPAC.

Dans la présente description, on entend, selon la convention IUPAC, par micropores les pores dont le diamètre est inférieur à 2 nm, c'est à dire 0,002 µm; par mésopores les pores dont le diamètre est supérieur ou égal à 2 nm, c'est à dire 0,002 µm et inférieur ou égal à 50 nm, c'est à dire 0,05 µm et par macropores les pores dont le diamètre est supérieur à 50 nm, c'est à dire 0,05 µm.

Le volume poreux total est mesuré par porosimétrie au mercure selon la norme ASTM D4284-92 avec un angle de mouillage de 140°, par exemple au moyen d'un appareil modèle Autopore III^{™} de la marque Microméritics^{™}.

La surface spécifique BET est mesurée par physisorption à l'azote selon la norme ASTM D3663-03, méthode décrite dans l'ouvrage Rouquerol F.; Rouquerol J.; Singh K. « Adsorption by Powders & Porous Solids: Principle, methodology and applications », Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On entend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the determination of crystallite size», J. I. Langford and A. J. C. Wilson.

La teneur en nickel et en cuivre est mesurée par fluorescence X.

Dans la présente description, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ». En outre, dans la présente description, le terme « sensiblement » correspondent à une approximation de ± 10%, préférablement de ± 5%, très préférablement de ± 2%, d'une valeur de référence telle qu'une distance, une vitesse, un débit, une teneur en composés, une température, une pression, etc.

### 2. Procédé de préparation du catalyseur

Les étapes dudit procédé de préparation sont décrites en détail ci-après.

### Etape a) Mise en contact du support avec un premier précurseur de nickel et un premier composé organique

Le dépôt du premier précurseur de nickel et du premier composé organique comprenant au moins une fonction acide carboxylique sur ledit support, conformément à la mise en œuvre de l'étape a), peut être réalisé par imprégnation, à sec ou en excès, ou encore par dépôt - précipitation, selon des méthodes bien connues de l'Homme du métier.

De manière préférée, ladite étape a) est réalisée par imprégnation à sec, laquelle consiste à mettre en contact le support du catalyseur avec une solution, contenant au moins le premier précurseur de nickel et au moins un premier composé organique comprenant une fonction aide carboxylique, dont le volume de la solution est compris entre 0,25 et 1,5 fois le volume poreux du support à imprégner.

Ladite étape a) est préférentiellement réalisée par imprégnation du support consistant par exemple en la mise en contact dudit support avec au moins une solution, aqueuse ou organique (par exemple le méthanol ou l'éthanol ou le phénol ou l'acétone ou le toluène ou le diméthylsulfoxyde (DMSO)) ou bien constituée d'un mélange d'eau et d'au moins un solvant organique, contenant au moins le premier précurseur de nickel au moins partiellement à l'état dissous et au moins un premier composé organique comprenant une fonction aide carboxylique, ou encore en la mise en contact dudit support avec au moins une solution colloïdale d'au moins un précurseur du nickel, sous forme oxydée (nanoparticules d'oxyde, d'oxy(hydroxyde) ou d'hydroxyde du nickel) ou sous forme réduite (nanoparticules métalliques du nickel à l'état réduit) et d'au moins un premier composé organique comprenant une fonction aide carboxylique. De préférence, la solution est aqueuse. Le pH de cette solution pourra être modifié par l'ajout éventuel d'un acide ou d'une base.

De manière préférée, ledit premier précurseur de nickel est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. De manière préférée, on utilise avantageusement comme premier précurseur de nickel, le nitrate de nickel, le chlorure de nickel, l'acétate de nickel ou le hydroxycarbonate de nickel. De manière très préférée, le premier précurseur de nickel est le nitrate de nickel.

La concentration en nickel en solution est ajustée selon le volume poreux du support encore disponible de façon à obtenir pour le catalyseur supporté, une teneur en nickel comprise entre 1 et 50 % poids en élément nickel par rapport au poids total du catalyseur, plus préférentiellement entre 2 et 40 % poids et encore plus préférentiellement entre 3 et 35 % poids et encore plus préférentiellement 5 et 28 % poids.

Ledit premier composé organique comprenant au moins une fonction acide carboxylique peut être un composé organique aliphatique, saturé ou insaturé, ou un composé organique aromatique. De préférence, le composé organique aliphatique, saturé ou insaturé, comprend entre 1 et 9 atomes de carbone, de préférence entre 2 et 7 atomes de carbone. De préférence, le composé organique aromatique comprend entre 7 et 10 atomes de carbone, de préférence entre 7 et 9 atomes de carbone.

Ledit premier composé organique aliphatique, saturé ou insaturé, ou ledit composé organique aromatique, comprenant au moins une fonction acide carboxylique peut être choisi parmi les acides monocarboxyliques, les acides dicarboxyliques, les acides tricarboxyliques, les acides tétracarboxyliques.

Avantageusement, le premier composé organique comprenant au moins une fonction acide carboxylique est choisi parmi l'acide éthanedioïque (acide oxalique), l'acide propanedioïque (acide malonique), l'acide pentanedioïque (acide glutarique), l'acide hydroxyacétique (acide glycolique), l'acide 2-hydroxypropanoïque (acide lactique), l'acide 2-hydroxypropanedioïque (acide tartronique), l'acide 2-hydroxypropane-1,2,3-tricarboxylique (acide citrique), l'acide 2,3-dihydroxybutanedioïque (acide tartrique), l'acide 2-oxopropanoïque (acide pyruvique), l'acide 4-oxopentanoïque (acide lévulinique).

Avantageusement, le rapport molaire entre le premier composé organique introduit à l'étape a) et l'élément nickel également introduit à l'étape a) est compris entre 0,01 et 5,0 mol/mol, de préférence entre 0,05 et 2,0 mol/mol, plus préférentiellement entre 0,1 et 1,5 mol/mol et encore plus préférentiellement entre 0,3 et 1,2 mol/mol.

### Etape b) Séchage et calcination

Le premier précurseur du catalyseur obtenu à l'issue de l'étape a) est ensuite séché à une température inférieure à 250°C, de préférence comprise entre 15°C et 180°C, plus préférentiellement entre 30°C et 160°C, encore plus préférentiellement entre 50°C et 150°C, et de manière encore plus préférentielle entre 70°C et 140°C, pendant une durée typiquement comprise entre 0,5 heure et 12 heures, et de façon plus préférée pendant une durée comprise entre 0,5 heure et 5 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

Après séchage, le premier précurseur de catalyseur séché est calciné à une température comprise entre 250°C et 600°C, de préférence entre 350°C et 550°C, pendant une durée typiquement comprise entre 0,5 et 24 heures, de façon préférée pendant une durée comprise entre 0,5 et 12 heures, et de façon encore plus préférée pendant une durée comprise entre 0,5 à 10 heures, de préférence sous une atmosphère inerte ou sous une atmosphère contenant de l'oxygène. Des durées plus longues ne sont pas exclues, mais n'apportent pas nécessairement d'amélioration.

### Etape c) Mise en contact du précurseur de catalyseur calciné avec un précurseur de cuivre, un deuxième précurseur de nickel et un second composé organique

Le dépôt du nickel, du cuivre et du second composé organique comprenant au moins une fonction acide carboxylique sur le précurseur de catalyseur calciné obtenu à l'issue de l'étape b) peut être réalisé par imprégnation, à sec ou en excès, ou encore par dépôt - précipitation, selon des méthodes bien connues de l'Homme du métier.

De manière préférée, ladite étape c) est réalisée par imprégnation à sec, laquelle consiste à mettre en contact le précurseur de catalyseur calciné avec une solution, comprenant, de préférence constituée de, au moins un précurseur du nickel, au moins un précurseur de cuivre, et au moins un second composé organique comprenant au moins une fonction acide carboxylique, dont le volume de la solution est compris entre 0,25 et 1,5 fois le volume poreux du support à imprégner.

Ladite étape c) est préférentiellement réalisée par imprégnation simultanée du précurseur de catalyseur calciné obtenu à l'issue de l'étape b) consistant par exemple en la mise en contact dudit précurseur de catalyseur calciné avec au moins une solution, aqueuse ou organique (par exemple le méthanol ou l'éthanol ou le phénol ou l'acétone ou le toluène ou le diméthylsulfoxyde (DMSO)) ou bien constituée d'un mélange d'eau et d'au moins un solvant organique, comprenant, de préférence étant constituée de, au moins un deuxième précurseur de nickel au moins partiellement à l'état dissous, au moins un précurseur de cuivre au moins partiellement à l'état dissous et un second composé organique comprenant au moins une fonction acide carboxylique, ou encore en la mise en contact dudit précurseur de catalyseur calciné avec au moins une solution colloïdale comprenant, de préférence étant constituée de, au moins un précurseur du nickel et d'un précurseur de cuivre sous forme oxydées (nanoparticules d'oxyde, d'oxy(hydroxyde) ou d'hydroxyde du nickel et de cuivre) ou sous forme réduites (nanoparticules métalliques du nickel et de cuivre à l'état réduit) et au moins un second composé organique comprenant au moins une fonction acide carboxylique. De préférence, la solution est aqueuse. Le pH de cette solution peut être modifié par l'ajout éventuel d'un acide ou d'une base.

De manière préférée, ledit deuxième précurseur de nickel et le précurseur de cuivre sont introduits en solution aqueuse.

Lorsque le deuxième précurseur de nickel est introduit en solution aqueuse, on utilise avantageusement le deuxième précurseur de nickel sous forme de nitrate, de carbonate, d'acétate, de chlorure, d'hydroxyde, d'hydroxycarbonate, d'oxalate, de sulfate, de formiate, de complexes formés par un polyacide ou un acide-alcool et ses sels, de complexes formés avec les acétylacétonates, de complexes tétrammine ou hexammine, ou encore de tout autre dérivé inorganique soluble en solution aqueuse, laquelle est mise en contact avec ledit précurseur de catalyseur. De manière préférée, on utilise avantageusement comme deuxième précurseur de nickel, le nitrate de nickel, l'hydroxyde de nickel, le carbonate de nickel, le chlorure de nickel, ou le hydroxycarbonate de nickel. De manière très préférée, le deuxième précurseur de nickel est le nitrate de nickel, le carbonate de nickel ou l'hydroxyde de nickel.

Lorsque le précurseur de cuivre est introduit en solution aqueuse, on utilise avantageusement un précurseur de cuivre sous forme minérale ou organique. Sous forme minérale, le précurseur de cuivre peut être choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre. De manière très préférée, le sel précurseur du cuivre est le nitrate de cuivre.

Le deuxième précurseur de nickel est avantageusement approvisionné à l'étape c) à une concentration voulue pour obtenir sur le catalyseur final (i.e. obtenu à l'issue de l'étape d) de séchage/calcination ou de l'étape e) de réduction si cette dernière est effectuée) une teneur comprise entre 0,5 et 10 % poids en élément nickel par rapport au poids total du catalyseur final, de préférence entre 0,5 et 8% en poids, plus préférentiellement entre 1 et 7% en poids, encore plus préférentiellement entre 1 et 5% en poids.

Les quantités du ou des précurseurs de cuivre introduites dans la solution selon l'étape c) sont choisies de telle manière que la teneur totale en cuivre est comprise entre 0,5 et 15 % en poids en élément cuivre par rapport au poids total du catalyseur final (i.e. obtenu à l'issue de l'étape d) de séchage/calcination ou de l'étape e) de réduction si cette dernière est effectuée), de préférence comprise entre 0,5 et 12 % poids, de manière préférée comprise entre 0,75 et 10 % poids, et encore plus préférentiellement entre 1 et 9% en poids.

Ledit second composé organique comprenant au moins une fonction acide carboxylique peut être un composé organique aliphatique, saturé ou insaturé, ou un composé organique aromatique. De préférence, le composé organique aliphatique, saturé ou insaturé, comprend entre 1 et 9 atomes de carbone, de préférence entre 2 et 7 atomes de carbone. De préférence, le composé organique aromatique comprend entre 7 et 10 atomes de carbone, de préférence entre 7 et 9 atomes de carbone.

Ledit second composé organique aliphatique, saturé ou insaturé, ou ledit composé organique aromatique, comprenant au moins une fonction acide carboxylique peut être choisi parmi les acides monocarboxyliques, les acides dicarboxyliques, les acides tricarboxyliques, les acides tétracarboxyliques.

Avantageusement, le second composé organique comprenant au moins une fonction acide carboxylique est choisi parmi l'acide éthanedioïque (acide oxalique), l'acide propanedioïque (acide malonique), l'acide pentanedioïque (acide glutarique), l'acide hydroxyacétique (acide glycolique), l'acide 2-hydroxypropanoïque (acide lactique), l'acide 2-hydroxypropanedioïque (acide tartronique), l'acide 2-hydroxypropane-1,2,3-tricarboxylique (acide citrique), l'acide 2,3-dihydroxybutanedioïque (acide tartrique), l'acide 2-oxopropanoïque (acide pyruvique), l'acide 4-oxopentanoïque (acide lévulinique).

Avantageusement, le rapport molaire entre le second composé organique introduit à l'étape c) et l'élément nickel également introduit à l'étape c) est compris entre 0,02 et 5 mol/mol, de préférence entre 0,1 et 3 mol/mol, plus préférentiellement entre 0,2 et 2 mol/mol et encore plus préférentiellement entre 0,3 et 2 mol/mol.

Avantageusement, le ratio entre le rapport molaire entre le second composé organique et le nickel introduit à l'étape c) et le rapport molaire entre le premier composé organique et le nickel introduit à l'étape a) est supérieur ou égal à 1,5, de préférence compris entre 2 et 5.

Avantageusement, le second composé organique introduit à l'étape c) est identique au premier composé organique introduite à l'étape a).

### Etape d) Séchage

Le second précurseur du catalyseur obtenu à l'issue de l'étape c) est ensuite séché à une température inférieure à 250°C, de préférence comprise entre 15°C et 180°C, plus préférentiellement entre 30°C et 160°C, encore plus préférentiellement entre 50°C et 150°C, et de manière encore plus préférentielle entre 70°C et 140°C, pendant une durée typiquement comprise entre 0,5 heure et 12 heures, et de façon plus préférée pendant une durée comprise entre 0,5 heure et 5 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

### Etape e) calcination (optionnelle)

Après séchage, le catalyseur obtenu à l'issue de l'étape d) est avantageusement calciné à une température comprise entre 250°C et 600°C, de préférence entre 350°C et 550°C, pendant une durée typiquement comprise entre 0,5 et 24 heures, de façon préférée pendant une durée comprise entre 0,5 et 12 heures, et de façon encore plus préférée pendant une durée comprise entre 0,5 à 10 heures, de préférence sous une atmosphère inerte ou sous une atmosphère contenant de l'oxygène. Des durées plus longues ne sont pas exclues, mais n'apportent pas nécessairement d'amélioration.

### Etape f) Réduction par un gaz réducteur (optionnelle)

Dans un mode de réalisation selon l'invention, préalablement à l'utilisation du catalyseur dans le réacteur catalytique et la mise en œuvre d'un procédé d'hydrogénation, on effectue une étape de traitement réducteur f) en présence d'un gaz réducteur de manière à obtenir un catalyseur comprenant du nickel au moins partiellement sous forme métallique. Cette étape est avantageusement réalisée *in-situ* c'est-à-dire après le chargement du catalyseur dans un réacteur d'hydrogénation. Ce traitement permet d'activer ledit catalyseur et de former des particules métalliques, en particulier du nickel à l'état zéro valent. La réalisation *in-situ* du traitement réducteur du catalyseur permet de s'affranchir d'une étape supplémentaire de passivation du catalyseur par un composé oxygéné ou par le CO₂, ce qui est nécessairement le cas lorsque le catalyseur est préparé en réalisant un traitement réducteur ex-situ, c'est-à-dire en dehors du réacteur utilisé pour l'hydrogénation de composés aromatiques ou polyaromatiques. En effet, lorsque le traitement réducteur est réalisé ex-situ, il est nécessaire de réaliser une étape de passivation afin de préserver la phase métallique du catalyseur en présence d'air (lors des opérations de transport et de chargement du catalyseur dans le réacteur d'hydrogénation), puis de réaliser une étape nouvelle étape de réduction du catalyseur.

Le gaz réducteur est de préférence l'hydrogène. L'hydrogène peut être utilisé pur ou en mélange (par exemple un mélange hydrogène/azote, hydrogène/argon, hydrogène/méthane). Dans le cas où l'hydrogène est utilisé en mélange, toutes les proportions sont envisageables.

Ledit traitement réducteur est réalisé à une température supérieure ou égale à 150°C et inférieure à 250°C, de préférence comprise entre 160 et 230°C, et plus préférentiellement entre 170 et 220°C. La durée du traitement réducteur est comprise entre 5 minutes et moins de 5 heures, de préférence entre 10 minutes et 4 heures, et encore plus préférentiellement entre 10 minutes et 210 minutes.

La présence de l'alliage de nickel-cuivre au moins partiellement sous forme réduite permet de recourir à des conditions opératoires de réduction de la phase active de nickel moins sévères que dans l'art antérieur et permet ainsi de réaliser directement l'étape de réduction au sein du réacteur dans lequel on souhaite réaliser l'hydrogénation de composés insaturés ou aromatiques.

Par ailleurs, la présence de cuivre dans le catalyseur permet de conserver une bonne activité du catalyseur et une bonne durée de vie du catalyseur lorsque ce dernier est mis en contact avec une charge hydrocarbonée comprenant du soufre. En effet, par rapport au nickel, le cuivre présent dans le catalyseur capte plus facilement les composés soufrés compris dans la charge, ce qui limite l'empoisonnement irréversible des sites actifs. La montée en température jusqu'à la température de réduction désirée est généralement lente, par exemple fixée entre 0,1 et 10°C/min, de préférence entre 0,3 et 7°C/min.

Le débit d'hydrogène, exprimé en L/heure/gramme de précurseur de catalyseur est compris entre 0,01 et 100 L/heure/gramme de catalyseur, de préférence entre 0,05 et 10 L/heure/gramme de précurseur de catalyseur, de façon encore plus préférée entre 0,1 et 5 L/heure/gramme de précurseur de catalyseur.

### 3. Catalyseur

Le procédé de préparation selon l'invention permet d'obtenir un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, la taille des particules de nickel dans le catalyseur, mesurée sous forme oxyde, étant inférieure à 5 nm.

De préférence, au moins une partie du nickel et du cuivre se présente sous la forme d'un alliage de nickel-cuivre, répondant avantageusement à la formule NiₓCu_{y} avec x compris entre 0,1 et 0,9 et y compris entre 0,1 et 0,9.

De préférence, la teneur en nickel comprise dans l'alliage cuivre-nickel est comprise entre 0,5 et 15% en poids en élément nickel par rapport au poids total du catalyseur, de préférence entre 1 et 12% en poids, et plus préférentiellement entre 1 et 10% en poids.

La taille des particules de nickel, mesurée sous forme oxyde, dans le catalyseur est inférieure à 5 nm, plus préférentiellement inférieure à 4 nm, et encore plus préférentiellement inférieure ou égale à 3 nm.

La teneur en nickel dans ledit catalyseur est avantageusement comprise entre 1 et 50 % poids par rapport au poids total du catalyseur, plus préférentiellement entre 2 et 40 % poids et encore plus préférentiellement entre 3 et 35 % poids et encore plus préférentiellement 5 et 25% poids par rapport au poids total du catalyseur

La teneur en cuivre est comprise entre 0,5 et 15 % en poids en élément cuivre par rapport au poids total du catalyseur, de préférence comprise entre 0,5 et 12 % poids, de manière préférée comprise entre 0,75 et 10 % poids, et encore plus préférentiellement entre 1 et 9 % en poids.

La surface spécifique du catalyseur est généralement comprise entre 10 m²/g et 350 m²/g, de préférence entre 25 m²/g et 300 m²/g, de façon plus préférée entre 40 m²/g et 250 m²/g.

Le volume poreux total du catalyseur est généralement compris entre 0,1 et 1 ml/g, de préférence compris entre 0,2 ml/g et 0,8 ml/g, et de manière particulièrement préférée compris entre 0,3 ml/g et 0,7 ml/g.

La phase active du catalyseur ne comprend préférablement pas de métal du groupe VIB. Elle ne comprend notamment pas de molybdène ou de tungstène.

Ledit catalyseur (et le support utilisé pour la préparation du catalyseur) est sous forme de grains ayant avantageusement un diamètre compris entre 0,5 et 10 mm. Les grains peuvent avoir toutes les formes connues de l'Homme du métier, par exemple la forme de billes (ayant de préférence un diamètre compris entre 1 et 8 mm), d'extrudés, de tablettes, de cylindres creux. De préférence, le catalyseur (et le support utilisé pour la préparation du catalyseur) sont sous forme d'extrudés de diamètre compris entre 0,5 et 10 mm, de préférence entre 0,8 et 3,2 mm et de manière très préférée entre 1,0 et 2,5 mm et de longueur comprise entre 0,5 et 20 mm. On entend par « diamètre » des extrudés le diamètre du cercle circonscrit à la section droite de ces extrudés. Le catalyseur peut être avantageusement présenté sous la forme d'extrudés cylindriques, multilobés, trilobés ou quadrilobés. De préférence sa forme sera trilobée ou quadrilobée. La forme des lobes pourra être ajustée selon toutes les méthodes connues de l'art antérieur.

### 4. Support

Les caractéristiques de l'alumine, mentionnées dans cette section, correspondent aux caractéristiques de l'alumine avant la réalisation de l'étape a) du procédé de préparation selon l'invention.

Le support est une alumine c'est-à-dire que le support comporte au moins 95%, de préférence au moins 98%, et de manière particulièrement préférée au moins 99% poids d'alumine par rapport au poids du support. L'alumine présente généralement une structure cristallographique du type alumine delta, gamma ou thêta, seule ou en mélange.

Le support d'alumine, peut comprendre des impuretés telles que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium, ou encore des métaux alcalins, de préférence le lithium, le sodium ou le potassium, et/ou les alcalino-terreux, de préférence le magnésium, le calcium, le strontium ou le baryum ou encore du soufre.

La surface spécifique de l'alumine est généralement comprise entre 10 m²/g et 400m²/g, de préférence entre 30 m²/g et 350 m²/g, de façon plus préférée entre 50 m²/g et 300m²/g.

Le volume poreux de l'alumine est généralement compris entre 0,1 ml/g et 1,2 ml/g, de préférence compris entre 0,3 ml/g et 0,9 ml/g, et de manière très préférée compris entre 0,5 ml/g et 0,9 ml/g.

Le diamètre médian mésoporeux est avantageusement compris entre 3 nm et 25 nm, et de préférence entre 6 et 20 nm, et de manière particulièrement préférée compris entre 8 et 18 nm.

### 5. Procédé d'hydrogénation sélective

La présente invention a également pour objet un procédé d'hydrogénation sélective de composés polyinsaturés contenant au moins 2 atomes de carbone par molécule, tels que les dioléfines et/ou les acétyléniques et/ou les alcénylaromatiques, aussi appelés styréniques, contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur obtenu par le procédé de préparation tel que décrit ci-avant dans la description.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la coupe C5+ (composés hydrocarbonés ayant au moins 5 atomes de carbone), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 300°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C2-C3 de vapocraquage, une coupe C3 de vapocraquage, une coupe C4 de vapocraquage, une coupe C5 de vapocraquage et une essence de vapocraquage encore appelée essence de pyrolyse ou coupe C5+.

La coupe C2 de vapocraquage, avantageusement utilisée pour la mise en œuvre du procédé d'hydrogénation sélective selon l'invention, présente par exemple la composition suivante : entre 40 et 95 % poids d'éthylène, de l'ordre de 0,1 à 5 % poids d'acétylène, le reste étant essentiellement de l'éthane et du méthane. Dans certaines coupes C2 de vapocraquage, entre 0,1 et 1 % poids de composés en C3 peut aussi être présent.

La coupe C3 de vapocraquage, avantageusement utilisée pour la mise en œuvre du procédé d'hydrogénation sélective selon l'invention, présente par exemple la composition moyenne suivante : de l'ordre de 90 % poids de propylène, de l'ordre de 1 à 8 % poids de propadiène et de méthylacétylène, le reste étant essentiellement du propane. Dans certaines coupes C3, entre 0,1 et 2 % poids de composés en C2 et de composés en C4 peut aussi être présent.

Une coupe C2 - C3 peut aussi être avantageusement utilisée pour la mise en œuvre du procédé d'hydrogénation sélective selon l'invention. Elle présente par exemple la composition suivante : de l'ordre de 0,1 à 5 % poids d'acétylène, de l'ordre de 0,1 à 3 % poids de propadiène et de méthylacétylène, de l'ordre de 30 % poids d'éthylène, de l'ordre de 5 % poids de propylène, le reste étant essentiellement du méthane, de l'éthane et du propane. Cette charge peut aussi contenir entre 0,1 et 2 % poids de composés en C4.

La coupe C4 de vapocraquage, avantageusement utilisée pour la mise en œuvre du procédé d'hydrogénation sélective selon l'invention, présente par exemple la composition massique moyenne suivante : 1 % poids de butane, 46,5 % poids de butène, 51 % poids de butadiène, 1,3 % poids de vinylacétylène et 0,2 % poids de butyne. Dans certaines coupes C4, entre 0,1 et 2 % poids de composés en C3 et de composés en C5 peut aussi être présent.

La coupe C5 de vapocraquage, avantageusement utilisée pour la mise en œuvre du procédé d'hydrogénation sélective selon l'invention, présente par exemple la composition suivante : 21 % poids de pentanes, 45 % poids de pentènes, 34 % poids de pentadiènes.

L'essence de vapocraquage ou essence de pyrolyse, avantageusement utilisée pour la mise en œuvre du procédé d'hydrogénation sélective selon l'invention, correspond à une coupe hydrocarbonée dont la température d'ébullition est généralement comprise entre 0 et 300°C, de préférence entre 10 et 250°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante : 5 à 30 % poids de composés saturés (paraffines et naphtènes), 40 à 80 % poids de composés aromatiques, 5 à 20 % poids de mono-oléfines, 5 à 40 % poids de dioléfines, 1 à 20 % poids de composés alcénylaromatiques, l'ensemble des composés formant 100 %. Elle contient également de 0 à 1000 ppm poids de soufre, de préférence de 0 à 500 ppm poids de soufre.

De manière préférée, la charge d'hydrocarbures polyinsaturés traitée conformément au procédé d'hydrogénation sélective selon l'invention est une coupe C2 de vapocraquage, ou une coupe C2-C3 de vapocraquage, ou une essence de vapocraquage.

Le procédé d'hydrogénation sélective selon l'invention vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation sélective des coupes C2, C2-C3, C3, C4, C5 et C5+ de vapocraquage peut être réalisée en phase gazeuse ou en phase liquide, de préférence en phase liquide pour les coupes C3, C4, C5 et C5+ et en phase gazeuse pour les coupes C2 et C2-C3. Une réaction en phase liquide permet d'abaisser le coût énergétique et d'augmenter la durée de cycle du catalyseur.

D'une manière générale, l'hydrogénation sélective d'une charge d'hydrocarbures contenant des composés polyinsaturés contenant au moins 2 atomes de carbone par molécule et ayant un point d'ébullition final inférieur ou égal à 300°C s'effectue à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire (définie comme le rapport du débit volumique de charge sur le volume du catalyseur) comprise entre 0,1 et 200 h⁻¹ pour un procédé réalisé en phase liquide, ou à un ratio molaire hydrogène/(composés polyinsaturés à hydrogéner) compris entre 0,5 et 1000 et à une vitesse volumique horaire comprise entre 100 et 40000 h⁻¹ pour un procédé réalisé en phase gazeuse.

Dans un mode de réalisation selon l'invention, lorsqu'on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 0,5 et 10, de préférence entre 0,7 et 5,0 et de manière encore plus préférée entre 1,0 et 2,0, la température est comprise entre 0 et 200°C, de préférence entre 20 et 200 °C et de manière encore plus préférée entre 30 et 180°C, la vitesse volumique horaire (V.V.H.) est comprise généralement entre 0,5 et 100 h⁻¹, de préférence entre 1 et 50 h⁻¹ et la pression est généralement comprise entre 0,3 et 8,0 MPa, de préférence entre 1,0 et 7,0 MPa et de manière encore plus préférée entre 1,5 et 4,0 MPa.

Plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 0,7 et 5,0, la température est comprise entre 20 et 200 °C, la vitesse volumique horaire (V.V.H.) est comprise généralement entre 1 et 50 h⁻¹ et la pression est comprise entre 1,0 et 7,0 MPa.

Encore plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 1,0 et 2,0, la température est comprise entre 30 et 180°C, la vitesse volumique horaire (V.V.H.) est comprise généralement entre 1 et 50 h⁻¹ et la pression est comprise entre 1,5 et 4,0 MPa.

Le débit d'hydrogène est ajusté afin d'en disposer en quantité suffisante pour hydrogéner théoriquement l'ensemble des composés polyinsaturés et de maintenir un excès d'hydrogène en sortie de réacteur.

Dans un autre mode de réalisation selon l'invention, lorsqu'on effectue un procédé d'hydrogénation sélective dans lequel la charge est une coupe C2 de vapocraquage et/ou une coupe C2-C3 de vapocraquage comportant des composés polyinsaturés, le ratio molaire (hydrogène)/(composés polyinsaturés à hydrogéner) est généralement compris entre 0,5 et 1000, de préférence entre 0,7 et 800, la température est comprise entre 0 et 300°C, de préférence entre 15 et 280 °C, la vitesse volumique horaire (V.V.H.) est comprise généralement entre 100 et 40000 h⁻¹, de préférence entre 500 et 30000 h⁻¹ et la pression est généralement comprise entre 0,1 et 6,0 MPa, de préférence entre 0,2 et 5,0 MPa.

### 6. Procédé d'hydrogénation des aromatiques

La présente invention a également pour objet un procédé d'hydrogénation d'au moins un composé aromatique ou polyaromatique contenu dans une charge d'hydrocarbures ayant un point d'ébullition final inférieur ou égal à 650°C, généralement entre 20 et 650°C, et de préférence entre 20 et 450°C. Ladite charge d'hydrocarbures contenant au moins un composé aromatique ou polyaromatique peut être choisi parmi les coupes pétrolières ou pétrochimiques suivantes : le reformat du reformage catalytique, le kérosène, le gazole léger, le gazole lourd, les distillats de craquage, tels que l'huile de recyclage de FCC, le gazole d'unité de cokéfaction, les distillats d'hydrocraquage.

La teneur en composés aromatiques ou polyaromatiques contenus dans la charge d'hydrocarbures traitée dans le procédé d'hydrogénation selon l'invention est généralement compris entre 0,1 et 80% en poids, de préférence entre 1 et 50% en poids, et de manière particulièrement préférée entre 2 et 35% en poids, le pourcentage étant basé sur le poids total de la charge d'hydrocarbures. Les composés aromatiques présents dans ladite charge d'hydrocarbures sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou de chlore, de préférence inférieure à 100 ppm poids, et de manière particulièrement préférée inférieure à 10 ppm poids.

La mise en œuvre technologique du procédé d'hydrogénation des composés aromatiques ou polyaromatiques est par exemple réalisée par injection, en courant ascendant ou descendant, de la charge d'hydrocarbures et de l'hydrogène dans au moins un réacteur à lit fixe. Ledit réacteur peut être de type isotherme ou de type adiabatique. Un réacteur adiabatique est préféré. La charge d'hydrocarbures peut avantageusement être diluée par une ou plusieurs ré-injection(s) de l'effluent, issu dudit réacteur où se produit la réaction d'hydrogénation des aromatiques, en divers points du réacteur, situés entre l'entrée et la sortie du réacteur afin de limiter le gradient de température dans le réacteur. La mise en œuvre technologique du procédé d'hydrogénation des aromatiques selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation des composés aromatiques ou polyaromatiques peut être réalisée en phase gazeuse ou en phase liquide, de préférence en phase liquide. D'une manière générale, l'hydrogénation des composés aromatiques ou polyaromatiques s'effectue à une température comprise entre 30 et 350°C, de préférence entre 50 et 325°C, à une pression comprise entre 0,1 et 20 MPa, de préférence entre 0,5 et 10 MPa, à un ratio molaire hydrogène/(composés aromatiques à hydrogéner) entre 0,1 et 10 et à une vitesse volumique horaire comprise entre 0,05 et 50 h⁻¹, de préférence entre 0,1 et 10 h⁻¹ d'une charge d'hydrocarbures contenant des composés aromatiques ou polyaromatiques et ayant un point d'ébullition final inférieur ou égal à 650°C, généralement entre 20 et 650°C, et de préférence entre 20 et 450°C.

Le débit d'hydrogène est ajusté afin d'en disposer en quantité suffisante pour hydrogéner théoriquement l'ensemble des composés aromatiques et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière du procédé selon l'invention, on réalise un procédé d'hydrogénation du benzène d'une charge d'hydrocarbures, tel que le reformat issu d'une unité de reformage catalytique. La teneur en benzène dans ladite charge d'hydrocarbures est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation du benzène contenu dans la charge d'hydrocarbures peut être réalisée en phase gazeuse ou en phase liquide, de préférence en phase liquide. Lorsqu'elle est réalisée en phase liquide, un solvant peut être présent, tel que le cyclohexane, l'heptane, l'octane. D'une manière générale, l'hydrogénation du benzène s'effectue à une température comprise entre 30 et 250°C, de préférence entre 50 et 200°C, et de manière plus préférée entre 80 et 180°C, à une pression comprise entre 0,1 et 10 MPa, de préférence entre 0,5 et 4 MPa, à un ratio molaire hydrogène/(benzène) entre 0,1 et 10 et à une vitesse volumique horaire comprise entre 0,05 et 50 h⁻¹, de préférence entre 0,5 et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention va maintenant être illustré via les exemples ci-après qui ne sont nullement limitatifs.

### Exemples

Pour tous les catalyseurs mentionnés dans les exemples mentionnées ci-après, le support est une alumine A présentant une surface spécifique de 180 m²/g, un volume poreux de 0,7 mL/g et un diamètre médian mésoporeux de 10 nm.

### Exemple 1 : Préparation d'une solution aqueuse S1 de premier précurseur de Ni avec un premier composé organique

La solution aqueuse S1 est préparée en dissolvant 58 g de nitrate de nickel (NiNO₃, fournisseur Strem Chemicals^{®}) et 14,35 g d'acide malonique (CAS 141-82-2 ; fournisseur Fluka^{®}) dans un volume de 42 mL d'eau distillée. La solution est chauffée à 60°C pour faciliter la dissolution du nitrate de nickel. Le ratio molaire additif/Ni est fixé à 0,4 mol/mol. On obtient la solution S1.

### Exemple 2 : Préparation d'une solution aqueuse S2 de précurseurs de l'alliage NiCu (5% en poids de Ni) mais sans second composé organique

La solution aqueuse de précurseurs de l'alliage NiCu (solution S2) utilisée pour la préparation des catalyseurs contenant du NiCu est préparée en dissolvant 14,5 g de nitrate de nickel (NiNO₃, fournisseur Strem Chemicals^{®}) dans un volume de 13 mL d'eau distillée. On obtient une solution dont la concentration en Ni est de 116,6 g de Ni par litre de solution. Le précurseur nitrate de cuivre est ensuite ajouté afin d'avoir un ratio molaire Ni/Cu de 1.

On obtient la solution S2. Elle permet d'introduire les précurseurs de l'alliage NiCu avec une teneur massique en Ni par rapport au catalyseur final de 5% poids par rapport au poids total du catalyseur.

### Exemple 3 : Préparation d'une solution aqueuse S3 des précurseurs de l'alliage NiCu et un second composé organique (5% en poids de Ni)

La solution aqueuse de précurseurs de l'alliage NiCu (solution S3) utilisée pour la préparation des catalyseurs contenant du NiCu est préparée en dissolvant 14,5 g de nitrate de nickel (NiNO₃, fournisseur Strem Chemicals^{®}) dans un volume de 13 mL d'eau distillée. On obtient une solution dont la concentration en Ni est de 116,6 g de Ni par litre de solution. Le précurseur nitrate de cuivre est ensuite ajouté afin d'avoir un ratio molaire Ni/Cu de 1. L'acide malonique est également ajouté à la solution afin d'avoir un ratio molaire additif/Ni est fixé à 0,90 mol/mol.

On obtient la solution S3. Elle permet d'introduire les précurseurs de l'alliage NiCu avec une teneur massique en Ni par rapport au catalyseur final de 5% poids par rapport au poids total du catalyseur.

### Exemple 4 : Préparation d'un catalyseur A (non conforme - pas de second composé organique)

10 g d'alumine A sont imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite 10 ml de la solution S2 préparée dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur A contenant 24% en poids de l'élément nickel par rapport au poids total du catalyseur (dont 5% en poids en élément nickel compris dans l'alliage NiCu). Les caractéristiques du catalyseur A ainsi obtenu sont reportées dans le tableau 1 ci-après.

### Exemple 5 : Préparation d'un catalyseur B (non conforme - pas de second composé organique)

10 g d'alumine A sont imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite ce catalyseur intermédiaire est ré-imprégné à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite 10 ml de la solution S2 préparée dans l'exemple 2 est imprégnée à sec en l'ajoutant goutte-à-goutte. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur B contenant 27% en poids de l'élément nickel par rapport au poids total du catalyseur (dont 5% en poids en élément nickel compris dans l'alliage NiCu). Les caractéristiques du catalyseur B ainsi obtenu sont reportées dans le tableau 1 ci-après.

### Exemple 6 : Préparation d'un catalyseur C (conforme)

10 g d'alumine A sont imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite 10 ml de la solution S3 préparée dans l'exemple 3 est imprégnée à sec en l'ajoutant goutte-à-goutte. Le ratio molaire entre le 2^{nd} composé organique/Ni (solution NiCu, solution S2) et le ratio molaire 1^{er} composé organique/Ni (Ni actif solution S1) est de 2,25. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur C contenant 24% en poids de l'élément nickel par rapport au poids total du catalyseur (dont 5% en poids en élément nickel compris dans l'alliage NiCu). Les caractéristiques du catalyseur C ainsi obtenu sont reportées dans le tableau 1 ci-après.

### Exemple 7 : Préparation d'un catalyseur D (conforme - double imprégnation)

10 g d'alumine A sont imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite ce catalyseur intermédiaire est ré-imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite 10 ml de la solution S3 préparée dans l'exemple 3 est imprégnée à sec en l'ajoutant goutte-à-goutte. e ratio molaire entre le 2^{nd} composé organique/Ni (solution NiCu, solution S2) et le ratio molaire 1^{er} composé organique/Ni (Ni actif solution S1) est de 2,25. Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur D contenant 27% en poids de l'élément nickel par rapport au poids total du catalyseur (dont 5% en poids en élément nickel compris dans l'alliage NiCu). Les caractéristiques du catalyseur D ainsi obtenu sont reportées dans le tableau 1 ci-après.

### Exemple 8 : Préparation d'un catalyseur E (non conforme - pas de NiCu et de second composé organique)

10 g d'alumine A sont imprégnés à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures. Ensuite ce catalyseur intermédiaire est ré-imprégné à sec avec 7,1 ml de la solution S1. Le précurseur de catalyseur obtenu est ensuite séché en étuve pendant 12 heures à 120°C puis calciné sous un flux d'air sec de 1 L/h/g de catalyseur à 450°C pendant 2 heures.

On obtient le catalyseur E contenant 22 % en poids de l'élément nickel par rapport au poids total du catalyseur. Les caractéristiques du catalyseur E ainsi obtenu sont reportées dans le tableau 1 ci-après.

**Tableau 1**

| Catalyseur | Second composé organique | Ni° (% pds)* | Taille des particules (nm) |
|---|---|---|---|
| A (non conforme) | non | 19 | 6 |
| B (non conforme) | non | 22 | 9 |
| C (conforme) | oui | 19 | 2,5 |
| D (conforme) | oui | 22 | 3 |
| E (non conforme) | non (pas de NiCu) | 22 | 5 |

| | | | |
|---|---|---|---|
| *Ni° hors alliage NiCu | | | |

### Exemple 9 : Caractérisation

La quantité d'alliage obtenue après l'étape de calcination puis réduction a été déterminée par analyse par diffraction des rayons X (DRX) sur des échantillons de catalyseur sous forme de poudre.

La quantité de nickel sous forme métallique obtenue après l'étape de réduction a été déterminée par analyse par diffraction des rayons X (DRX) sur des échantillons de catalyseur sous forme de poudre. Entre l'étape de réduction et pendant toute la durée de la caractérisation par DRX les catalyseurs ne sont jamais remis à l'air libre. Les diagrammes de diffraction sont obtenus par analyse radiocristallographique au moyen d'un diffractomètre en utilisant la méthode classique des poudres avec le rayonnement Kα1 du cuivre (λ = 1,5406 Â).

Le taux de réduction a été calculé en calculant l'aire de la raie de Ni⁰ située vers 52°2θ, sur l'ensemble des diffractogrammes de chaque échantillon de catalyseur analysé, puis en soustrayant le signal présent dès la température ambiante sous la raie à 52° et qui est dû à l'alumine.

La tableau 2 ci-après rassemble les taux de réduction ou encore la teneur en nickel métallique Ni° (exprimée en % poids par rapport au poids total de Ni « actif » qui ne compose pas l'alliage) pour tous les catalyseurs A à E caractérisés par DRX après une étape de réduction à 170°C pendant 190 minutes sous flux d'hydrogène. Ces valeurs ont également été comparées avec le taux de réduction obtenu pour le catalyseur E (Ni seul) après une étape de réduction classique (c'est-à-dire à une température de 400°C pendant 15 heures sous flux d'hydrogène).

A température ambiante sur tous les catalyseurs, après calcination, contenant du cuivre et du nickel, nous détectons de l'alumine sous forme delta et thêta, et des grandes raies de NiO et de CuO.

Nous détectons par ailleurs après réduction une raie correspondant à l'alliage sous forme Ni_{0,76}Cu_{0,24}.

Afin d'évaluer le taux de réductibilité et donc la formation du Ni⁰, on mesure l'aire de la raie de Ni⁰ située vers 52°2θ, sur l'ensemble des diffractogrammes, en soustrayant le signal présent dès la température ambiante sous la raie à 52° et qui est dû à l'alumine. On peut ainsi déterminer le pourcentage relatif de Ni⁰ cristallisé après la réduction.

**Tableau 2**

| Catalyseur | Réduction finale | Ni/Cu | Taille des particules (nm) | Pourcentage de Ni° seul (DRX) après réduction (%) |
|---|---|---|---|---|
| A (non conforme) | 170°C, 190 min | oui | 6 | 90 |
| B (non conforme) | 170°C, 190 min | oui | 9 | 92 |
| C (conforme) | 170°C,190 min | oui | 2,5 | 90 |
| D (conforme) | 170°C, 190 min | oui | 3 | 90 |
| E (non conforme) | 170°C, 190 min | non | 4 | 0* |
| E (non conforme) | 400°C, 15 h | non | 4 | 80 |

| | | | | |
|---|---|---|---|---|
| *Nickel sous forme de NiO | | | | |

Pour le catalyseur E (22%Ni seul/alumine), le taux de réductibilité de nickel est de 0% après exactement le même traitement de réduction sous hydrogène que pour les catalyseurs A, B, C, D. Il faut réduire à 400°C le catalyseur E pour avoir une réduction du nickel oxyde en Ni° de l'ordre de 80%.

### Exemple 10 : Tests catalytiques : performances en hydrogénation sélective d'un mélange contenant du styrène et de l'isoprène (A_{HYD1})

Les catalyseurs A à E décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 % pds styrène (fournisseur Sigma Aldrich^{®}, pureté 99%), 8 % pds isoprène (fournisseur Sigma Aldrich^{®}, pureté 99%), 84 % pds n-heptane (solvant) (fournisseur VWR^{®}, pureté > 99% chromanorm HPLC). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Dans un autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170 °C pendant 90 minutes sous un flux d'hydrogène de 1 L/h/g (rampe de montée en température de 1 °C/min) pour les catalyseurs A à E (ce qui correspond ici à l'étape f) du procédé de préparation selon l'invention selon un mode de réalisation). Ensuite l'autoclave est porté à la température du test égale à 30°C. Au temps t=0, environ 30 g d'un mélange contenant du styrène, de l'isoprène, du n-heptane, du pentanethiol et du thiophène sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

Un autre test a été effectué pour le catalyseur E, mais avec une température de réduction du catalyseur de 400°C pendant 15 heures.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à E sont reportées dans le tableau 3 ci-après. Elles sont rapportées à l'activité catalytique (A_{HYD1}) mesurée pour le catalyseur E préparé dans les conditions classiques de réduction (à une température de 400°C pendant 15 heures sous flux d'hydrogène).

### Exemple 11 : Tests catalytiques : performances en hydrogénation du toluène (A_{HYD2})

Les catalyseurs A à E décrits dans les exemples ci-dessus sont également testés vis-à-vis de la réaction d'hydrogénation du toluène. La réaction d'hydrogénation sélective est opérée dans le même autoclave que celui décrit à l'exemple 10.

Dans un autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170 °C pendant 90 minutes sous un flux d'hydrogène de 1 L/h/g (rampe de montée en température de 1 °C/min) pour les catalyseurs A à E (ce qui correspond ici à l'étape f) du procédé de préparation selon l'invention selon un mode de réalisation). Après l'ajout de 216 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS^{®}, pureté > 99,8%) sont introduits dans l'autoclave (la composition initiale du mélange réactionnel est alors toluène 6 % pds / n-heptane 94 % pds) et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à E sont reportées dans le tableau 3 ci-après. Elles sont rapportées à l'activité catalytique (A_{HYD2}) mesurée pour le catalyseur E préparé dans les conditions classiques de réduction (à une température de 400°C pendant 15 heures sous flux d'hydrogène).

**Tableau 3**

| Catalyseur | Teneur en Ni (%) | AHYD1 (%) | A_{HYD2} (%) |
|---|---|---|---|
| A (non conforme) | 27 | 75 | 75 |
| B (non conforme) | 27 | 76 | 80 |
| C (conforme) | 24 | 160 | 170 |
| D (conforme) | 27 | 180 | 195 |
| E (non conforme) | 22 | <1 | <1 |
| E (non conforme) | 22 | 100 | 100 |

Ces résultats montrent bien une amélioration significative des performances en A_{HYD1} et A_{HYD2} des catalyseurs C et D obtenus par le procédé de préparation selon l'invention, par rapport aux catalyseurs A, B et E non conformes. Pour les catalyseurs A et B, le nickel oxyde est réduit à 170°C à hauteur de 90% et présente des particules qui ont grossi lors de l'étape d'imprégnation du NiCu sans présence du second composé organique. Le catalyseur E est en retrait d'activité du fait de l'absence de NiCu et donc d'une réductibilité du NiO à 170°C quasi nulle. Les catalyseurs C et D conservent de petites particules de nickel du fait de l'ajout d'acide malonique (second composé organique) lors de l'ajout du NiCu en post-traitement par rapport à l'ajout du précurseur de la phase active de nickel.

## Revendications

1. Procédé de préparation d'un catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, la taille des particules de nickel dans le catalyseur, mesurée sous forme oxyde, étant inférieure à 5 nm, lequel procédé comprend au moins les étapes suivantes :
a) on met en contact le support d'alumine avec au moins une solution contenant au moins un premier précurseur de nickel et au moins un premier composé organique comprenant au moins une fonction acide carboxylique pour obtenir un premier précurseur de catalyseur ;
b) on sèche le premier précurseur de catalyseur obtenu à l'issue de l'étape a) à une température inférieure à 250°C puis on calcine le premier précurseur de catalyseur séché à une température comprise entre 250°C et 550°C pour obtenir un précurseur de catalyseur calciné ;
c) on met en contact le précurseur de catalyseur calciné obtenu à l'issue de l'étape b) avec au moins une solution contenant au moins un deuxième précurseur de nickel, au moins un précurseur de cuivre et au moins un second composé organique comprenant au moins une fonction acide carboxylique pour obtenir un second précurseur de catalyseur ;
d) on sèche le second précurseur de catalyseur obtenu à l'issue de l'étape c) à une température inférieure à 250°C.

2. Procédé selon la revendication 1, dans lequel le rapport molaire entre ledit composé organique introduit à l'étape a) et l'élément nickel également introduit à l'étape a) est compris entre 0,01 et 5,0 mol/mol.

3. Procédé selon l'une des revendications 1 ou 2, dans lequel le rapport molaire entre ledit composé organique introduit à l'étape c) et l'élément nickel également introduit à l'étape c) est compris entre 0,02 et 5 mol/mol.

4. Procédé selon l'une quelconque des revendications 1 à 3, dans lequel le rapport molaire entre le nickel introduit lors des étapes a) et c) et le cuivre introduit lors de l'étape c) est compris entre 0,5 et 5 mol/mol.

5. Procédé selon l'une quelconque des revendications 1 à 4, dans lequel le ratio entre le rapport molaire entre le second composé organique et le nickel introduit à l'étape c) et le rapport molaire entre le premier composé organique et le nickel introduit à l'étape a) est supérieur à 1,5.

6. Procédé selon l'une quelconque des revendications 1 à 5, dans lequel les étapes a) et b) sont réalisées au moins deux fois avant de réaliser l'étape c).

7. Procédé selon l'une quelconque des revendications 1 à 6, dans lequel le premier composé organique de l'étape a) et le second composé organique de l'étape c) sont choisis parmi l'acide oxalique, l'acide malonique, l'acide glycolique, l'acide acide lactique, l'acide tartronique, l'acide citrique, l'acide tartrique, l'acide pyruvique, l'acide lévulinique.

8. Procédé selon l'une quelconque des revendications 1 à 7, dans lequel le premier composé organique de l'étape a) et le second composé organique de l'étape c) sont identiques.

9. Procédé selon l'une quelconque des revendications 1 à 8, dans lequel le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre et le fluorure de cuivre.

10. Procédé selon l'une quelconque des revendications 1 à 9, dans lequel le premier précurseur et/ou le deuxième précurseur de nickel est le nitrate de nickel, le chlorure de nickel, l'acétate de nickel ou l'hydroxycarbonate de nickel.

11. Procédé selon l'une quelconque des revendications 1 à 10, comprenant en outre une étape e) dans laquelle on calcine le catalyseur obtenu à l'issue de l'étape d) à une température comprise entre 250°C et 550°C.

12. Procédé selon l'une quelconque des revendications 1 à 11, comprenant en outre une étape f) dans laquelle on réduit le catalyseur obtenu à l'issue de l'étape d), optionnellement obtenu à l'issue de l'étape e), par mise en contact dudit catalyseur avec un gaz réducteur à une température supérieure ou égale à 150°C et inférieure à 250°C.

13. Catalyseur obtenu par le procédé selon l'une quelconque des revendications 1 à 12, ledit catalyseur comprenant du nickel et du cuivre, à raison de 1 et 50 % en poids en élément nickel par rapport au poids total du catalyseur, et à raison de 0,5 à 15 % en poids en élément cuivre par rapport au poids total du catalyseur, et un support poreux d'alumine, la taille des particules de nickel dans le catalyseur, mesurée sous forme oxyde, étant inférieure à 5 nm.

14. Procédé d'hydrogénation sélective de composés polyinsaturés contenant au moins 2 atomes de carbone par molécule contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur selon la revendication 13 ou obtenu selon le procédé de préparation selon l'une quelconque des revendications 1 à 12.

15. Procédé d'hydrogénation d'au moins un composé aromatique ou polyaromatique contenu dans une charge d'hydrocarbures ayant un point d'ébullition final inférieur ou égal à 650°C, ledit procédé étant réalisé en phase gazeuse ou en phase liquide, à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire comprise entre 0,05 et 50 h⁻¹, en présence d'un catalyseur selon la revendication 13 ou obtenu selon le procédé de préparation selon l'une quelconque des revendications 1 à 12.

## Patentansprüche

1. Verfahren zur Herstellung eines Katalysators, der Nickel und Kupfer in einer Menge von 1 und 50 Gew.-% des Elements Nickel, bezogen auf das Gesamtgewicht des Katalysators, und in einer Menge von 0,5 bis 15 Gew.-% des Elements Kupfer, bezogen auf das Gesamtgewicht des Katalysators, und einen porösen Aluminiumoxid-Träger umfasst, die Größe der Nickelteilchen im Katalysator, die in der Oxidform gemessen wird, weniger als 5 nm beträgt, wobei das Verfahren mindestens die folgenden Schritte umfasst:
a) man bringt den Aluminiumoxid-Träger mit mindestens einer Lösung, die mindestens eine erste Nickelvorstufe und mindestens eine erste organische Verbindung mit mindestens einer Carbonsäurefunktion enthält, in Kontakt, wobei man eine erste Katalysatorvorstufe erhält;
b) man trocknet die am Ende von Schritt a) erhaltene erste Katalysatorvorstufe bei einer Temperatur von weniger als 250 °C und calciniert die getrocknete erste Katalysatorvorstufe dann bei einer Temperatur zwischen 250°C und 550 °C, wobei man eine calcinierte Katalysatorvorstufe erhält;
c) man bringt die am Ende von Schritt b) erhaltene calcinierte Katalysatorvorstufe mit mindestens einer Lösung, die mindestens eine zweite Nickelvorstufe, mindestens eine Kupfervorstufe und mindestens eine zweite organische Verbindung mit mindestens einer Carbonsäurefunktion enthält, in Kontakt, wobei man eine zweite Katalysatorvorstufe erhält;
d) man trocknet die am Ende von Schritt c) erhaltene zweite Katalysatorvorstufe bei einer Temperatur von weniger als 250 °C.

2. Verfahren nach Anspruch 1, bei dem das Molverhältnis zwischen der in Schritt a) eingetragenen organischen Verbindung und dem ebenfalls in Schritt a) eingetragenen Element Nickel zwischen 0,01 und 5,0 mol/mol liegt.

3. Verfahren nach einem der Ansprüche 1 oder 2, bei dem das Molverhältnis zwischen der in Schritt c) eingetragenen organischen Verbindung und dem ebenfalls in Schritt c) eingetragenen Element Nickel zwischen 0,02 und 5 mol/mol liegt.

4. Verfahren nach einem der Ansprüche 1 bis 3, bei dem das Molverhältnis zwischen dem in den Schritten a) und c) eingetragenen Nickel und dem in Schritt c) eingetragenen Kupfer zwischen 0,5 und 5 mol/mol liegt.

5. Verfahren nach einem der Ansprüche 1 bis 4, bei dem das Verhältnis zwischen dem Molverhältnis zwischen der zweiten organischen Verbindung und dem in Schritt c) eingetragenen Nickel und dem Molverhältnis zwischen der ersten organischen Verbindung und dem in Schritt a) eingetragenen Nickel größer als 1,5 ist.

6. Verfahren nach einem der Ansprüche 1 bis 5, bei dem die Schritte a) und b) mindestens zweimal durchgeführt werden, bevor Schritt c) durchgeführt wird.

7. Verfahren nach einem der Ansprüche 1 bis 6, bei dem die erste organische Verbindung von Schritt a) und die zweite organische Verbindung von Schritt c) aus Oxalsäure, Malonsäure, Glykolsäure, Milchsäure, Tartronsäure, Citronensäure, Weinsäure, Brenztraubensäure und Lävulinsäure ausgewählt werden.

8. Verfahren nach einem der Ansprüche 1 bis 7, bei dem die erste organische Verbindung von Schritt a) und die zweite organische Verbindung von Schritt c) identisch sind.

9. Verfahren nach einem der Ansprüche 1 bis 8, bei dem die Kupfervorstufe aus Kupferacetat, Kupferacetylacetonat, Kupfernitrat, Kupfersulfat, Kupferchlorid, Kupferbromid, Kupferiodid und Kupferfluorid ausgewählt wird.

10. Verfahren nach einem der Ansprüche 1 bis 9, bei dem es sich bei der ersten Nickelvorstufe und/oder der zweiten Nickelvorstufe um Nickelnitrat, Nickelchlorid, Nickelacetat oder Nickelhydroxycarbonat handelt.

11. Verfahren nach einem der Ansprüche 1 bis 10, das außerdem einen Schritt e) umfasst, bei dem man den am Ende von Schritt d) erhaltenen Katalysator bei einer Temperatur zwischen 250 °C und 550 °C calciniert.

12. Verfahren nach einem der Ansprüche 1 bis 11, das außerdem einen Schritt f) umfasst, bei dem man den am Ende von Schritt d) oder gegebenenfalls am Ende von Schritt e) erhaltenen Katalysator durch Inkontaktbringen des Katalysators mit einem reduzierenden Gas bei einer Temperatur von mehr als oder gleich 150 °C und weniger als 250 °C reduziert.

13. Katalysator, erhalten durch das Verfahren nach einem der Ansprüche 1 bis 12, wobei der Katalysator Nickel und Kupfer in einer Menge von 1 und 50 Gew.-% des Elements Nickel, bezogen auf das Gesamtgewicht des Katalysators, und in einer Menge von 0,5 bis 15 Gew.-% des Elements Kupfer, bezogen auf das Gesamtgewicht des Katalysators, und einen porösen Aluminiumoxid-Träger umfasst, die Größe der Nickelteilchen im Katalysator, die in der Oxidform gemessen wird, weniger als 5 nm beträgt.

14. Verfahren zur selektiven Hydrierung von mehrfach ungesättigten Verbindungen mit mindestens 2 Kohlenstoffatomen pro Molekül, die in einem Kohlenwasserstoff-Einsatzstoff mit einem Siedeende kleiner oder gleich 300 °C enthalten sind, wobei das Verfahren bei einer Temperatur zwischen 0 und 300 °C, bei einem Druck zwischen 0,1 und 10 MPa, bei einem Molverhältnis von Wasserstoff zu mehrfach ungesättigten Verbindungen, die zu hydrieren sind, zwischen 0,1 und 10 und bei einer Katalysatorbelastung zwischen 0,1 und 200 h⁻¹ bei Durchführung des Verfahrens in der Flüssigphase oder bei einem Molverhältnis von Wasserstoff zu mehrfach ungesättigten Verbindungen, die zu hydrieren sind, zwischen 0,5 und 1000 und bei einer Katalysatorbelastung zwischen 100 und 40.000 h⁻¹ bei Durchführung des Verfahrens in der Gasphase in Gegenwart eines Katalysators nach Anspruch 13 oder eines nach dem Herstellungsverfahren nach einem der Ansprüche 1 bis 12 erhaltenen Katalysators durchgeführt wird.

15. Verfahren zur Hydrierung mindestens einer aromatischen oder polyaromatischen Verbindung, die in einem Kohlenwasserstoff-Einsatzstoff mit einem Siedeende kleiner oder gleich 650 °C enthalten ist, wobei das Verfahren in der Gasphase oder in der Flüssigphase, bei einer Temperatur zwischen 30 und 350 °C, bei einem Druck zwischen 0,1 und 20 MPa, bei einem Molverhältnis von Wasserstoff zu aromatischen Verbindungen, die zu hydrieren sind, zwischen 0,1 und 10 und bei einer Katalysatorbelastung zwischen 0,05 und 50 h⁻¹ in Gegenwart eines Katalysators nach Anspruch 13 oder eines nach dem Herstellungsverfahren nach einem der Ansprüche 1 bis 12 erhaltenen Katalysators durchgeführt wird.

## Claims

1. Process for preparing a catalyst comprising nickel and copper, in a proportion of 1% and 50% by weight of nickel element relative to the total weight of the catalyst, and in a proportion of 0.5% to 15% by weight of copper element relative to the total weight of the catalyst, and a porous alumina support, the size of the nickel particles in the catalyst, measured in oxide form, being less than 5 nm, which process comprises at least the following steps:
a) the alumina support is brought into contact with at least one solution containing at least one first nickel precursor and at least one first organic compound comprising at least one carboxylic acid function to obtain a first catalyst precursor;
b) the first catalyst precursor obtained at the end of step a) is dried at a temperature of less than 250°C and then the dried first catalyst precursor is calcined at a temperature of between 250°C and 550°C to obtain a calcined catalyst precursor;
c) the calcined catalyst precursor obtained at the end of step b) is brought into contact with at least one solution containing at least one second nickel precursor, at least one copper precursor and at least one second organic compound comprising at least one carboxylic acid function to obtain a second catalyst precursor;
d) the second catalyst precursor obtained at the end of step c) is dried at a temperature of less than 250°C.

2. Process according to Claim 1, wherein the mole ratio between said organic compound introduced in step a) and the nickel element also introduced in step a) is between 0.01 and 5.0 mol/mol.

3. Process according to either of Claims 1 and 2, wherein the mole ratio between said organic compound introduced in step c) and the nickel element also introduced in step c) is between 0.02 and 5 mol/mol.

4. Process according to any one of Claims 1 to 3, wherein the mole ratio between the nickel introduced during steps a) and c) and the copper introduced in step c) is between 0.5 and 5 mol/mol.

5. Process according to any one of Claims 1 to 4, wherein the ratio between the mole ratio between the second organic compound and the nickel introduced in step c) and the mole ratio between the first organic compound and the nickel introduced in step a) is greater than 1.5.

6. Process according to any one of Claims 1 to 5, wherein steps a) and b) are carried out at least twice before carrying out step c).

7. Process according to any one of Claims 1 to 6, wherein the first organic compound of step a) and the second organic compound of step c) are chosen from oxalic acid, malonic acid, glycolic acid, lactic acid, tartronic acid, citric acid, tartaric acid, pyruvic acid and levulinic acid.

8. Process according to any one of Claims 1 to 7, wherein the first organic compound of step a) and the second organic compound of step c) are identical.

9. Process according to any one of Claims 1 to 8, wherein the copper precursor is chosen from copper acetate, copper acetylacetonate, copper nitrate, copper sulfate, copper chloride, copper bromide, copper iodide and copper fluoride.

10. Process according to any one of Claims 1 to 9, wherein the first nickel precursor and/or the second nickel precursor are/is nickel nitrate, nickel chloride, nickel acetate or nickel hydroxycarbonate.

11. Process according to any one of Claims 1 to 10, further comprising a step e) wherein the catalyst obtained at the end of step d) is calcined at a temperature of between 250°C and 550°C.

12. Process according to any one of Claims 1 to 11, further comprising a step f) wherein the catalyst obtained at the end of step d), optionally obtained at the end of step e), is reduced by bringing said catalyst into contact with a reducing gas at a temperature of greater than or equal to 150°C and less than 250°C.

13. Catalyst obtained via the process according to any one of Claims 1 to 12, said catalyst comprising nickel and copper, in a proportion of 1% and 50% by weight of nickel element relative to the total weight of the catalyst, and in a proportion of 0.5% to 15% by weight of copper element relative to the total weight of the catalyst, and a porous alumina support, the size of the nickel particles in the catalyst, measured in oxide form, being less than 5 nm.

14. Process for the selective hydrogenation of polyunsaturated compounds containing at least 2 carbon atoms per molecule, contained in a hydrocarbon feedstock having a final boiling point below or equal to 300°C, which process being carried out at a temperature of between 0°C and 300°C, at a pressure of between 0.1 and 10 MPa, at a hydrogen/(polyunsaturated compounds to be hydrogenated) mole ratio of between 0.1 and 10 and at an hourly space velocity of between 0.1 and 200 h⁻¹ when the process is carried out in the liquid phase, or at a hydrogen/(polyunsaturated compounds to be hydrogenated) mole ratio of between 0.5 and 1000 and at an hourly space velocity of between 100 and 40 000 h⁻¹ when the process is carried out in the gas phase, in the presence of a catalyst according to Claim 13 or obtained according to the preparation process according to any one of Claims 1 to 12.

15. Process for the hydrogenation of at least one aromatic or polyaromatic compound contained in a hydrocarbon feedstock having a final boiling point below or equal to 650°C, said process being carried out in the gas phase or in the liquid phase, at a temperature of between 30 and 350°C, at a pressure of between 0.1 and 20 MPa, at a hydrogen/(aromatic compounds to be hydrogenated) mole ratio of between 0.1 and 10 and at an hourly space velocity of between 0.05 and 50 h⁻¹, in the presence of a catalyst according to Claim 13 or obtained according to the preparation process according to any one of Claims 1 to 12.
